Europäisches Patentamt

European Patent Office

Office européen des brevets

(11) Veröffentlichungsnummer: **0 337 166**
**A2**

(12) # EUROPÄISCHE PATENTANMELDUNG

(21) Anmeldenummer: 89105070.0

(22) Anmeldetag: 21.03.89

(51) Int. Cl.⁴: **A61H 23/00 , B60N 1/00**

(30) Priorität: 11.04.88 HU 181288

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **Boczán, János, Dr. Med.**
**Dévai u. 22-26**
**H-1134 Budapest(HU)**

(72) Erfinder: **Boczán, János, Dr. Med.**
**Dévai u. 22-26**
**H-1134 Budapest(HU)**

(74) Vertreter: **Patentanwälte Viering & Jentschura**
**Steinsdorfstrasse 6**
**D-8000 München 22(DE)**

(54) **Verfahren zum Nachweisen, Vermindern bzw. Eliminieren der Müdigkeit von Fahrzeugfahrern sowie Kraftfahrzeug zum Durchführen des Verfahrens.**

(57) Verfahren zum Nachweisen, Vermindern bzw. Eliminieren der Müdigkeit von Fahrzeugfahrern sowie Kraftfahrzeug zum Durchführen des Verfahrens. Gemäß der Erfindung ist in die Rückenlehne des Fahrersitzes des Kraftfahrzeugs eine Ultraschallarmatur eingebaut, die an einen mit niedriger Frequenz betriebenen Ultraschallgeber anschließbar ist.

EP 0 337 166 A2

## Verfahren zum Nachweisen, Vermindern bzw. Eliminieren der Müdigkeit von Fahrzeugfahrern sowie Kraftfahrzeug zum Durchführen des Verfahrens.

Die Erfindung bezieht sich auf ein Verfahren zum Nachweisen, Vermindern bzw. Eliminieren der Müdigkeit von Fahrzeugfahrern sowie auf ein Kraftfahrzeug zum Durchführen des Verfahrens.

Es ist bekannt, daß beim Reisen bzw. Fahrzeugfahren bei den meisten Menschen anfangs ein positives Gefühlsmotiv entsteht, welches mit erhöhter Bewegungstendenz, mit lauterem Sprechen zum Ausdruck kommt. Dieser erhöhte Bereitschaftszustand, dieses Reisefieber kann auch bei Gruppenreisen erkannt werden, und nicht nur bei den Fahrgästen, sondern auch bei Fahrzeugfahrern.

Gegen das Ende der Arbeitsschicht, in den letzten zwei bis drei Stunden sind die Fahrzeugfahrer mehr reizbar, oft kommen negative Gefühlsmotive in den Vordergrund, ihre Bewegung ist langsamer, ihr Fahren ist sportlicher.

Die mit dem Fahrzeugfahren verbundene oder dadurch verursachte Müdigkeit wurde bereits in zahlreichen Formen geprüft, aber der Sachverhalt der Müdigkeit konnte nicht nachgewiesen werden. Durch die Publikationen und Studien der Abteilung für Verkehrsgesundheitswesen und Verkehrspsychologie des ungarischen Instituts für Verkehrswesen, in denen über die ausländische Literatur dieses Gebietes hinaus auch ihre eigenen Meßdaten und Analysen ausführlich dargestellt sind, ist es eindeutig anerkannt, daß sich die üblichen Methoden nicht bewährt haben.

Durch die supermechanisierten Kraftfahrzeuge ist aber der Komfort des Menschen nur teilweise erhöht, aus anderem Gesichtspunkt erscheinen sie als gesundheitsschädliche Faktoren und hier soll man nicht nur an die sekundäre Folgen der Bewegungsarmut denken. Es sollten derartige Kraftfahrzeuge erzeugt werden, die den biologischen Bedingungen des Menschen am meisten entsprechen. In den letzten Zeiten wurde die Messung der Wirkung des Fahrzeugfahrens ermöglicht. Mit dieser Technik kann die positive und negative Wirkung des Kraftfahrzeuges in der Funktion des hinterlassenen Wegs und der Fahrtzeit festgestellt werden. Das System ist aus ergonomischen (Kraftfahrzeugentwicklung), arbeitssanitären (Arbeitsschutz) und Verkehrssicherheitsgesichtspunkten bedeutend und dient zur Registrierung des Tons des vegetativen Nervensystems. Das Prüfsystem besteht aus den folgenden:

- Infrafernsehgerät, Komputer, zur Messung erforderliche Zubehöre.
- Zur automatischen Messung des vegetativen Indexes erforderliche Mittel (Ultraschall-Blutdruckmesser, Pulsmesser, integrierter Stromkreis, Anzeiger).
- Registrierung der Muskelspannung der Genickstrecke des Rückens. Die Komputeraufarbeitung eines am oberen hinteren Drittel des Kopfes und am Dornansatz des siebten Halswirbels, oder in dessen Umgebung angeordneten Bildes.

Es hat sich eindeutig bewiesen, daß der am Beginn der Fahrt wahrnehmbare, im Vergleich zum ausgeruhten Zustand erhöhte Bereitschaftszustand und der Müdigkeitszustand mit der Veränderung der Infrarotstrahlung der Haut verbunden ist. Die Veränderung ist am größten in dem sog. endarteriolösen System, wie z.B. die Blutversorgungsgebiete der Nase, der Fingerspitzen. Es hat sich auch herausgestellt, daß die Tonveränderung des vegetativen Nervensystems nämlich, ob eine sympathische oder parasympathische Dominanz besteht in der Infrarotstrahlung der Haut gut wahrzunehmen ist.

Es ist weiterhin bekannt, daß in der Heilkunde die Bordács'sche Behandlung mit pulsierendem Magneten in immer breiterem Kreis verwendet wird. Deren Wesen besteht darin, daß ein für diesen Zweck gefertigter Elektromagnet mittels in vorbestimmten Perioden verwendeten Stromunterbrechungen neben anderen vorbestimmten Parametern ein abgebrochenes magnetisches Feld zustandebringt. Die mit dem auf diesem Prinzip funktionierenden Gerät durchgeführte Behandlung wird als Behandlung mit pulsierendem Magnet bezeichnet.

Zur Zeit werden mehrere Tausend derartige Geräte mit Erfolg verwendet. Wie bekannt, ist das vegetative Nervensystem der Dirigent des allgemeinen und lokalen Stoffwechsels des menschlichen Organismus. Das vegetative Nervensystem besteht aus zwei voneinander aus anatomischem, biologischem, biochemischem Gesichtspunkt gut trennbaren, einander gegenüberstehenden Systemen. Im Fall des Gleichgewichtes dieser Systeme ist unser Gemeingefühl gut, unser Leben harmonisch. Dieses Nervensystem regelt, steuert die Eingeweide und die Drüsen, beziehungsweise deren Stoffwechsel so, daß es die darin laufenden Adern, die in der Aderwand befindliche glatte Muskulatur regelt. Mit der Tonerhöhung oder Erschlaffung der glatten Muskulatur verengen oder erweitern sich die Adern. Dementsprechend erhält das entsprechende Gebiet mehr oder weniger Nährstoff, Blut. Die Regelung des Aufbaus oder des Abbaus erfolgt abhängig davon. Das System funktioniert auf den verschiedenen Gebieten des Organismus oft dermaßen gegeneinander, daß an der einen Stelle die sympathische, an der anderen Stelle die parasympathische Wirkung dominant ist. Das ist der Schlüssel zum Verstehen des Funktionierens. Das Ziel ist das Aufrechterhalten des Organismus, und dies erfolgt beinahe vollständig unabhängig von unserem Willen.

Aufgrund der obigen Ausführungen wurde es erforderlich, eine derartige Methode auszuarbeiten, die unter Berücksichtigung der obigen Gesichtspunkte eine derartige komplexe Lösung repräsentiert, die die Messung und Verminderung der Müdigkeit einander zugeordnet durchführt.

Im Sinne der Erfindung erfüllen wir das gesetzte Ziel mit einem Verfahren bzw. ein Kraftfahrzeug, welches dadurch gekennzeichnet werden kann, daß der Fahrzeugfahrer während des Fahrens mit Hilfe einer in der Rückenlehne des Fahrersitzes eingebauten elektrischen Armatur einer Ultraschallbehandlung niedriger Frequenz unterworfen wird.

Im einzelnen ist dies in den Patentansprüchen angegeben.

Wir haben gefunden, daß wenn mit Hilfe eines Infrarotfernsehgerätes und eines Bildmagnetophons die Anzeichen der durch das Fahrzeugfahren verursachten Müdigkeit, die mit der Erwärmung der Eichelröhre, und bei Müdigkeit mit deren verminderten Infrarotstrahlung verbunden sind, fortlaufend registriert werden, die Müdigkeitsphase nach Zurücklegen von 360 km bei Autobusfahrern sich gemeldet hat. Während des Fahrens hat der Fahrzeugfahrer mit Hilfe der in der Rückenlehne des Fahrersitzes eingebauten Scheibe 15 Minuten lang eine Behandlung niedriger Frequenz erhalten. Nach der Behandlung haben sich die Anzeichen der Müdigkeit nicht gemeldet. Außerhalb der Versuche konnte mit der Veränderung der Infrarotstrahlung der Haut auch der Zustand objektiviert werden: der Fahrzeugfahrer äußerte sich über angenehmes Gemeingefühl, an den Thermogrammen war eine dem ausgeruhten Zustand entsprechende Veränderung wahrzunehmen.

Die Wirkung dieser Einrichtung auf den Menschen besteht in der Erhöhung oder Verminderung des Stoffwechsels, abhängig davon, mit welcher Frequenz die Pulsation eingestellt wurde. Dadurch kann die Ausbreitung oder Verengung der Ader, die Entspannung oder die Erhöhung der Spannung der Muskel erreicht werden. In den Versuchen wurde seit einem Jahr auch das Infrafernsehgerät verwendet, weiterhin hat auch die Erfindung als Grundmessung ihre Verwendung gefunden.

Wir haben mit dem Infrafernsehgerät außer mit Kranken auch mit gesunden Menschen Messungen durchgeführt. Neben den anderen elektrophysiologischen Prüfungen hat es sich erwiesen, daß eine Behandlung von 15-20 Minuten bei gesunden Menschen auch nach 6 Stunden ihre Wirkung auf die behandelte Person hatte. Diese Wirkung offenbart sich neben der erhöhten Infrarotstrahlung darin, daß sich die Kleinadern (Arteriolen-Endarteriolen) nicht verengen und der Blutstrom lebhafter bleibt.

So stellte sich die Frage, ob die Beschädigung, die in erster Linie in den Kleinadern erscheint, bei der Vibrationswirkung ausgesetzten Personen mit dieser Behandlung eliminiert werden kann. (Hier reden wir nicht über die Hochfrequenz-Wirkung der Vibration, wie zum Beispiel die den Knochen beschädigende Wirkung des Luftdruckhammers).

Mit der beschriebenen Methode haben wir bewiesen, daß bei den Fahrzeugfahrern in der Funktion des hinterlassenen Weges und der Fahrtzeit eine Gefäßverengungsphase auftritt. Diese Gefäßverengungsphase schafft eine aus dem Unfallgesichtspunkt heraus bedeutende Gefahrenlage und bei Personen in diesem Zustand kommen Unfälle häufiger vor. Dieses Stadium entspricht dem Müdigkeitszustand.

Wir haben die obenerwähnte Behandlung in der Sommerzeit bei unter besonders hoher Belastung stehenden, in erschöpftem Zustand Dienst leistenden Fahrzeugfahrern verwendet. Die mit Thermographie, Psyche-Teste, Reaktionszeitmessung mit Komputer durchgeführte Prüfung hat ein ziemlich gutes Ergebnis gezeigt. Wir haben festgestellt, daß sich nicht nur das Gemeingefühl, sondern auch die meßbaren Geistesfunktionen verbessert haben.

Wir haben das pulsierende Magnetfeld in den Fahrersitz eingebaut. Das auf Spannung von 24 V umgeänderte Gerät wurde - zu Versuchszwecken - in der nahen Vergangenheit auch in einem zerlegten Bussitz eingebaut.

Einrichtungen zur Entwicklung der Verkehrsmittel -verschiedene Kraftfahrzeuge, Arbeitsmaschinen, Flugzeuge, Eisenbahnen, Schiffe usw. - zur Weitererhöhung des Komforts des Menschen werden in zahlreichen Formen verwendet. Die wirtschaftlichen Kosten zur Entwicklung der Sitze, des Apparatekastens, der Federung, der Mittel zur Verminderung der Geräusch- und Vibrationswirkungen haben oft einen Betrag von mehreren Hunderttausend Dollar oder Mark. Die Komputer werden im alltäglichen Gebrauch der persönlichen Verkehrsmittel immer mehr verwendet, zum Beispiel der neben dem Lenkrad des Kraftfahrzeuges "Porsche" eingebaute Monitor sieht den Weg auch im Dunkeln, im Staub und Nebel, der Komputer steuert die richtige Benzinzufuhr, den richtigen Einschlagwinkel der Räder usw.

## Ansprüche

1. Verfahren zum Nachweisen, Vermindern bzw. Eliminieren der Müdigkeit von Fahrzeugfahrern, dadurch gekennzeichnet, daß der Fahrzeugfahrer während des Fahrens mit Hilfe einer in der Rückenlehne des Fahrersitzes eingebauten elektrischen Armatur einer Ultraschallbehandlung niedriger Frequenz unterworfen wird.

2. Verfahren nach Anspruch 1, dadurch gekennzeichnet, daß die Behandlung 10 bis 20 Minuten lang, zweckmäßig 15 Minuten lang durchgeführt wird.

3. Verfahren naoh Anspruch 1 oder 2, dadurch gekennzeichnet, daß das Einschalten der Behandlung in der Funktion des hinterlassenen Wegs vom Willen des Fahrzeugfahrers unabhängig erfolgt.

4. Kraftfahrzeug, bei dem in der Rückenlehne des Fahrersitzes eine Ultraschallarmatur eingebaut ist, die an einen mit niedriger Frequenz betriebenen Ultraschallgeber anschließbar ist.

5. Kraftfahrzeug nach Anspruch 4, bei dem die Ultraschallarmatur über einen Zeitgeber für eine Einschaltzeit von 10 bis 20 Minuten, vorzugsweise von 15 Minuten, betreibbar ist.

6. Kraftfahrzeug nach Anspruch 4 oder 5, bei dem die Ultraschallarmatur unabhängig vom Willen des Fahrzeugfahrers in Abhängigkeit von der zurückgelegten Fahrtstrecke einschaltbar ist.